(19) Europäisches Patentamt
European Patent Office
Office européen des brevets

(11) **EP 3 634 214 B1**

(12) **EUROPEAN PATENT SPECIFICATION**

(45) Date of publication and mention
of the grant of the patent:
**28.07.2021  Bulletin 2021/30**

(21) Application number: **18813314.4**

(22) Date of filing: **06.06.2018**

(51) Int Cl.:
*A61B 5/00* (2006.01)      *A61B 5/145* (2006.01)

(86) International application number:
**PCT/NO2018/050148**

(87) International publication number:
**WO 2018/226104 (13.12.2018 Gazette 2018/50)**

(54) **INTERSTITIAL FLUID OSMOTIC PRESSURE MEASURING DEVICE SYSTEM AND METHOD**

VORRICHTUNG UND VERFAHREN ZUR MESSUNG DES OSMOTISCHEN DRUCKS VON INTERSTITIELLER FLÜSSIGKEIT

DISPOSITIF, SYSTÈME ET PROCÉDÉ DE MESURE DE PRESSION OSMOTIQUE DE FLUIDE INTERSTITIEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **07.06.2017  NO 20170927**

(43) Date of publication of application:
**15.04.2020  Bulletin 2020/16**

(73) Proprietor: **Lifecare AS
5152 Bønes (NO)**

(72) Inventors:
• **FRISVOLD, Rune
  5224 Nesttun (NO)**
• **PFÜTZNER, Andreas
  55130 Mainz (DE)**

(74) Representative: **Zacco Norway AS
P.O. Box 2003 Vika
0125 Oslo (NO)**

(56) References cited:
**EP-A1- 2 055 226        US-A- 4 822 336
US-A1- 2010 056 888**

• **Erik Johannessen ET AL: "Toward an Injectable Continuous Osmotic Glucose Sensor", Journal of Diabetes Science and Technology Volume Diabetes Technology Society, 1 July 2010 (2010-07-01), XP55594748, Retrieved from the Internet: URL:https://www.ncbi.nlm.nih.gov/pmc/artic les/PMC2909520/pdf/dst-04-0882.pdf [retrieved on 2019-06-06]**
• **OLGA KRUSHINITSKAYA ET AL: "The assessment of potentially interfering metabolites and dietary components in blood using an osmotic glucose sensor based on the concanavalin Adextran affinity assay", BIOSENSORS AND BIOELECTRONICS, ELSEVIER SCIENCE LTD. UK, AMSTERDAM, NL, vol. 28, no. 1, 9 July 2011 (2011-07-09), pages 195-203, XP028340772, ISSN: 0956-5663, DOI: 10.1016/J.BIOS.2011.07.019 [retrieved on 2011-07-20]**
• **KRIEFTEWIRTH M ET AL: "Affinity sensor based on a membrane osmometer", TM - TECHNISCHES MESSEN/PLATTFORM FÜR METHODEN, SYSTEME UND ANWENDUNGEN DER MESSTECHNIK, R.OLDENBOURG VERLAG. MUNCHEN, DE, vol. 71, no. 1, 1 January 2004 (2004-01-01), pages 29-33, XP008092164, ISSN: 0171-8096, DOI: 10.1524/TEME.71.1.29.25425**

EP 3 634 214 B1

- SCHULTZ J S ET AL: "AFFINITY SENSOR: A NEW TECHNIQUE FOR DEVELOPING IMPLANTABLE SENSORSFOR GLUCOSE AND OTHER METABOLITES", 1 January 1990 (1990-01-01), SELECTED PAPERS ON OPTICAL FIBERS IN MEDICINE; [SPIE MILESTONE SERIES], BELLINGHAM, SPIE, US, PAGE(S) 358 - 366, XP000467825,

## Description

TECHNICAL FIELD

The present invention relates to a device, a system and a method for in- vivo measurement of augmented osmotic pressure. More specifically it relates to a double chamber sensor, one with an active solution and a membrane porous to glucose.

BACKGROUND

Commonly glucose measurement is based on manual point sample sensors instrumentation (finger-pricking). However, devices capable of conducting continuous blood glucose measurements would provide the most complete picture of the glucose variations during the course of the day and prevent the onset of dangerous events by for example trigger an alarm function when the blood glucose moves beyond what are considered safe levels. This is especially important when persons are sleeping or not being able to look after themselves. Although the continuous blood glucose measurement instrumentation is considered as the most effective method of monitoring glucose, the transcutaneous nature of the sensor patches, combined with limited sensor lifetimes and long start-up periods, has meant that the single use sensor for manual point sampling remains the most common.

There are some drawbacks to the manual point sampling method. The persons often experience pain and discomfort with the manual point sample devices, which might in turn compromise such self-testing regimes. Incomplete numbers of measurements taken during the course of a day may result in that the average person with diabetes spending periods during the days in a hyperglycaemic or hypoglycaemia state. Both these conditions are potentially dangerous and can contribute to vascular damage, mental confusion and even death.

The benefits of existing continuous sensing technologies come with major drawbacks and disadvantages, and hence there are currently no real commercial alternatives to the manual point sample method. Existing continuous glucose measurements technologies systems are inconvenient, complicated and costly. There are no alarm functions or digital memory for storing the data. The existing systems also have a limited operational lifetime and require frequent calibrations using external point sample meters.

The principle of the sensor according to the present invention is based on osmosis. In its simplest form, osmosis is the transport of a solvent across a semipermeable membrane caused by differences in the concentration of solutes on either side of the membrane. Osmosis is a process where certain kinds of molecules in a liquid are preferentially blocked by a "semipermeable" membrane. The solvent, which in some prior art has been water, is diffusing through the membrane into the more concentrated solution, more so than in the opposite direction. The result is a combination of two effects. One is that an osmotic pressure is built up in the volume of higher concentration. The other is the reduction in the concentration difference caused by the increased volume of solvent.

Ultimately, a dynamic equilibrium is reached, in which the increase in chemical potential caused by the osmotic pressure difference, equals the corresponding change caused by the difference in concentration. At osmotic equilibrium, the chemical potential of the solvent must equate the chemical potential of the pure solvent.

Detecting glucose by the principle of osmotic pressure holds promise of a glucose sensing technology that is suitable for both miniaturisation and long term continuous monitoring *in vivo* without causing patient discomfort or reducing quality of life.

An osmotic sensor for measuring blood glucose is described in the PhD Thesis "Osmotic sensor for blood glucose monitoring applications", by Olga Krushinitskaya, Department of Micro- and Nanosystems Technology, Vestfold University College, August 2012. The project in this PhD work addressed the technological aspect of developing a novel glucose sensor that was capable of tracking glucose continuously through the recording of osmotic pressure, based on the principle of utilizing the diffusion of water down its own concentration gradient, which enables an inherently simple sensor design in which the generated pressure is a function of the glucose concentration.

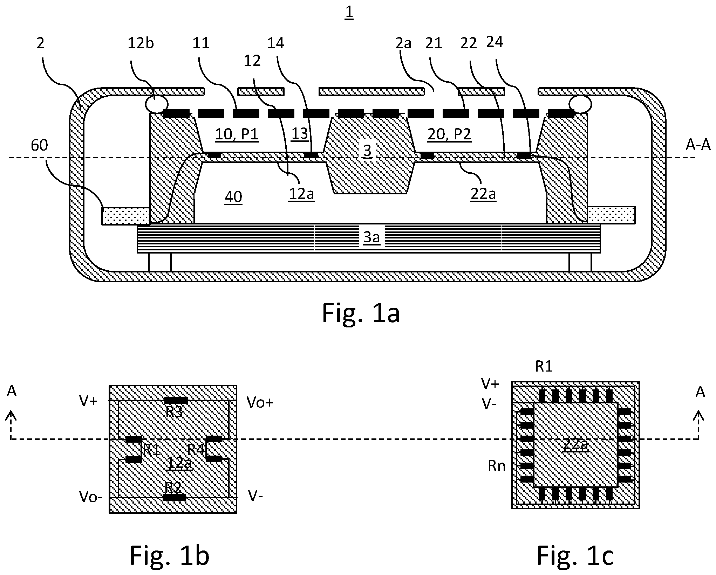

The osmotic sensor developed in the said project was based on the osmotic pressure generated by the competitive bonding between the sugar binding lectin Concanavalin A (ConA) and the long chained polysaccharide dextran, which forms a large macromolecular complex. Lectins are a group of proteins that have special binding sites for carbohydrates, and the ConA attaches strongly to glucose. The studies in the above thesis exploited the osmotic effect generated by the competitive bonding of ConA and dextran in the presence of glucose. As the concentration of glucose is increased, more of the larger ConA-dextran macromolecular complexes are split up into the smaller ConA-glucose and free dextran "sub units". In this manner the number of free particles inside the sensor is increased as a function of glucose, leading to a corresponding rise in the osmotic pressure, see Figure. 1A-B.

This process is reversible and as the glucose concentrations falls, the Con A reattaches back to the dextran forming a large macromolecular complex from the Con A and dextran "sub units". The corresponding decrease in the number of free particles triggers the osmotic pressure to fall.

Various types of membranes are known, having accurately dimensioned perforations which allow the passage of water, ions, lactates, but not of glucose. Such membranes are said to be semi-permeable. The diameter of the perforations ranges in this case, from about 0.6 nm to about 0.74 nm.

Semi-permeable membranes are also known, which have perforations larger than the molecules of glucose, thereby allowing the passage of glucose. By choosing such a membrane with perforations having a diameter in the order of 0.9 nm, a membrane can be obtained which is said to be at the limit of permeability to glucose.

Granted European patent EP 1 631 187 B1 discloses a sensor for *in vivo* measurement of osmotic changes. The sensor is an invasive sensor which can be implanted subcutaneously, and specially an invasive sensor comprising at least one differential pressure-transducer that measures the pressure difference between two fluid volumes confined by, in one end the at least one differential pressure-transducer, and in the other end osmotic membranes.

The sensor described in EP 1 631 187 B1 can be utilized to monitor any changes within the in chemistry *in vivo.* The type of solutes and their concentration observed *in vivo* gives a tremendous amount of information regarding the physiology of the body, and its condition. By measuring the composition for instance in the interstitial fluid (ISF), a lot of information can be obtained regarding de-hydration of the body and different diseases: diabetes, kidney functions, etc. Also normal variations for instance in lactate concentrations caused by physical activity can be monitored.

In addition to the substances mentioned above, which can change the osmolality in the body, one can also find substances which by medication give an osmotic contribution in the body fluid.

Measurement of glucose in ISF is becoming recognized as an alternative to measuring the glucose directly in the blood. The glucose measurement in blood is associated with several drawbacks. It needs a sample of blood, drawn from the body. Even though the equipment has become more sensitive, and therefore requires less blood, the process is associated with pain and the number of tests typically limited to less than 10 per day. It is also known that large variations in measured values can be caused by the measurement procedure.

In order to overcome problems related to hydrostatic pressure variations and their influence on the measurements obtained from a single chamber device, various solutions have been implemented where a second reference chamber is used for measuring hydrostatic pressure, where the glucose value can be found from the differential pressure between the chambers, where the second chamber has a membrane not permeable for glucose.

US patent 4,822,336 discloses a miniaturised implantable dual chamber device enclosed by two identical semi-permeable membranes permeable to glucose but impermeable to larger molecules and body cells. Both chambers are equipped with pressure transducers (or optional $CO_2$ transducers) and are filled with isotonic suspensions with similar osmotic strength as the peritoneal liquid from which the device is indirectly measuring the blood glucose. One of the chambers is equipped with a suspension of yeast cells which produce $CO_2$ at a rate which varies according to the concentration of glucose in the blood, and which is translated into corresponding pressure changes by the generated $CO_2$. The second chamber acts as a controlling device subtracting any pressure induced signals (such as motion) that may affect both chambers. The device is further equipped with a thermostat regulated heater that maintains a temperature between 37 and 41 °C in order to optimise the $CO_2$ producing capabilities of yeast and hence improve the sensitivity and response time of the sensor.

U.S. Pat. No. 5,337,747 discloses a micro fabricated sensor that comprises two separate measuring devices located in parallel. Both make use of semi-permeable membranes where one device measures the osmotic pressure from all particles that are larger, including glucose, whereas the second measures the osmotic activity from all particles larger than glucose. The differential pressure measurement between these two assigns the contribution from glucose alone. Each device incorporates semi-permeable membranes that are integrated with a silicon support structure which maintains stability of the membrane.

International patent application WP2009/025563 A1 discloses an apparatus and a method for measuring augmented osmotic pressure in a reference cavity. A semi-permeable membrane is used coupled to a transducer device capable of sensing mechanical induced bulging in the semi-permeable membrane, due to augmented osmotic pressure in the reference cavity. A second reference cavity is also proposed, where a solid membrane with an integrated pressure transducer covers the cavity and measures hydrostatic pressure. Osmotic pressure is then the difference between the output from the first transducer and the output of the second transducer, where hydrostatic pressure is eliminated.

SHORT SUMMARY

The invasive sensor technologies described above provides some measures for determining glucose-dependent osmotic pressure based on differential measurements. However, it remains challenging to give responsive in-vivo estimates of glucose concentrations due to the relatively small amplitude of the glucose-dependent osmotic pressure response compared to the large interference signals resulting from changes in hydrostatic pressure.

The scope of the present invention is to provide an improved estimate of the glucose-dependant signal response by improved disturbance rejection of pressure interference and noise, as well as improved robustness to sensor drift due to environmental changes.

A device in accordance with the invention is defined in claim 1.

In an embodiment the invention is a glucose measuring system comprising an interstitial fluid glucose measuring device as described above and an external device, wherein the interstitial fluid glucose measuring device and the external device both have wireless interfaces, and are arranged to communicate wirelessly.

A method in accordance with the invention is defined in claim 7.

The invention provides an improved estimate of the glucose-dependant signal response by offering improved disturbance rejection of pressure interference and noise, as well as robustness to sensor drift due to environmental changes. By combining the measurements from two pressure sensors, together with the improved glucose response due to the active solution in the reference chamber, the effect of the interfering pressure signals can be reduced and the estimate of the glucose value improved.

## BRIEF DESCRIPTION OF THE DRAWINGS

Fig. 1 shows a cross section of an interstitial fluid osmotic pressure measuring device according to an embodiment of the invention.

Fig. 2 illustrates the principle of using an active solution for increasing pressure in a chamber behind a glucose permeable membrane.

Fig. 3 illustrates a dual chamber sensor in use, where osmotic pressure in the first chamber increases as a result of glucose competing with polysaccharide to combine with lectin.

Fig. 4 shows a cross section of an interstitial fluid osmotic pressure measuring device according to an embodiment of the invention.

Fig. 5a shows an exploded view of the device in Fig. 4, while Fig. 5b shows a detail of the same embodiment.

Fig 6 Viscosity as a function of glucose concentration for dextran 10kDa, 40kDa and 70kDa. ConA concentration is equal to 3mM, dextran concentration is 0.5mM. Please note the $\gamma$-logarithmic scale.

Fig. 7 Viscosity as a function of glucose concentration for dextran 10kDa, 40kDa and 70kDa. ConA concentration is equal to 1.5 mM, dextran concentration is 0.5 mM. Please note the $\gamma$-logarithmic scale.

Fig. 8 Viscosity as a function of glucose concentration for dextran 10kDa, 40kDa and 70kDa. ConA concentration is equal to 1 mM, dextran concentration is 1 mM. Please note the $\gamma$-logarithmic scale.

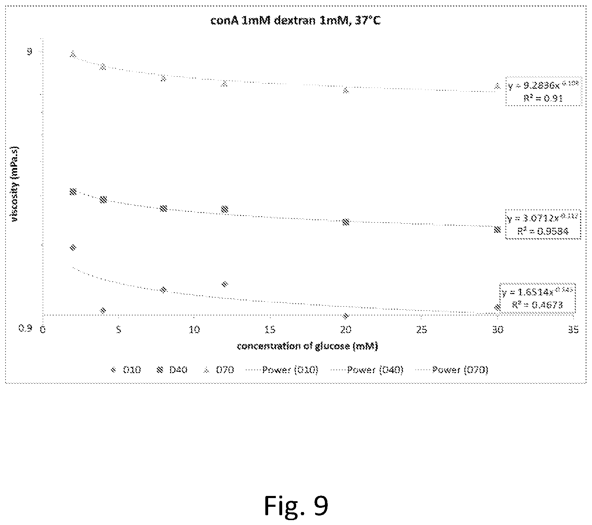

Fig. 9 Viscosity as a function of glucose concentration for two dextrans, 40kDa and 70k. ConA and dextran concentrations are equal to 1.5 mM.

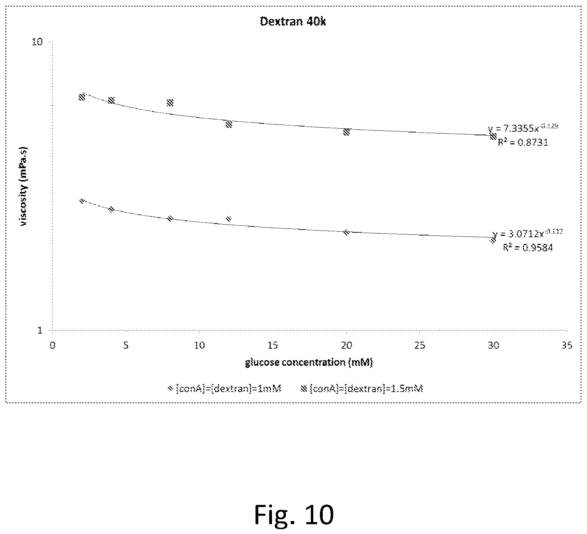

Fig. 10 Viscosity as a function of glucose concentration for dextran 40kDa, with two different ConA and dextran concentrations (1 and 1.5 mM).

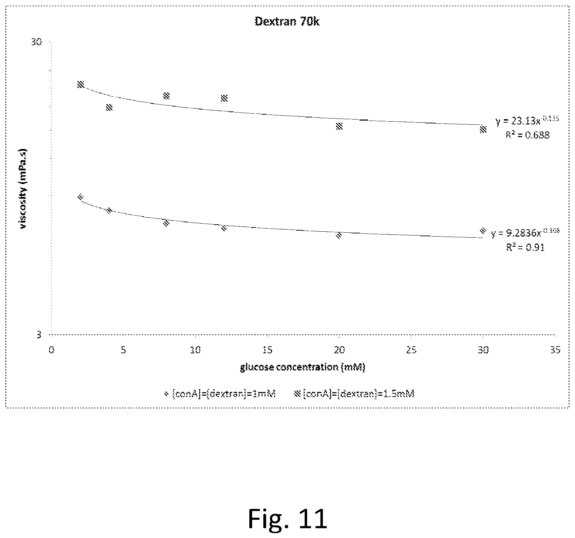

Fig. 11 Viscosity as a function of glucose concentration for dextran 70kDa, with two different ConA and dextran concentrations (1 and 1.5 mM).

Fig. 12 An example curve showing changes between 2 mM glucose solution and 30 mM glucose solution. Note the strong fast spike followed by a slow drift in the opposite direction to reach a stable equilibrium.

## EMBODIMENTS OF THE INVENTION

In the following description, various examples and embodiments of the invention are set forth in order to provide the skilled person with a more thorough understanding of the invention. The specific details described in the context of the various embodiments and with reference to the attached drawings are not intended to be construed as limitations. Rather, the scope of the invention is defined in the appended claims.

In the following the structural elements of the invention will be explained, while aspects of the active composition used in the first cavity will be explained thereafter. The combination of the two results in a positive synergetic effect as explained in the short summary section.

The structural elements of the invention will be explained with reference to an embodiment of the invention illustrated in the cross section of Fig. 1a. This embodiment illustrates the principle of operation and could have several different implementations, depending on the technology chosen for manufacturing.

Sensor structure

The interstitial fluid osmotic pressure measuring device (1) comprises a first cavity (10) and a second cavity (20), both configured for being in fluid communication with interstitial fluid outside the cavities.

Further, the device comprises first and second pressure sensors (12, 22), are configured for sensing the pressure in said first and second cavities (10, 20), respectively.

The first cavity (10) comprises an active solution that is a composition comprising a lectin and a polysaccharide.

A first glucose porous membrane (11) allowing glucose molecules to flow through the membrane in both directions, but preventing the larger lectin and polysaccharide molecules to leave the first cavity (10), defines a part of the cavity. In the Fig. the membrane is in the form of a flat lid on top of the cavity, but other shapes and implementations are possible as long as a fluid connection between the interstitial fluid and the cavity for exist for glucose molecules. As explained above, the osmotic pressure inside the cavities will increase when the number of glucose molecules entering the cavity increases.

The device (1) further comprises first and second pressure sensors (12, 22), arranged to sense a first and a second pressure (P1, P2) in the first and second cavity (10, 20), respectively. The pressure sensor output will reflect the change in osmolality in the two chambers. The pressure sensors will typically be pressure transducers able to sense a mechanical deflection as a result of varying pressure.

In an embodiment, the first pressure sensor (12) is arranged opposite the first glucose porous membrane (11) in the first cavity (10) and/or the second pressure sensor (22) is arranged opposite the second glucose porous membrane (21) in the secondcavity (10).

In this embodiment the device (1) comprises a vacuum chamber (40), with a fluid having a pressure level below ambient pressure, and the first and second pressure sensors (12, 22), are arranged to sense the first and the second pressure (P1, P2) with respect to the pressure in the vacuum chamber (40). The pressure measurements are therefore absolute pressure measurements with a fixed reference.

The pressure sensors (12, 22) in Fig. 1 have a solid, non-porous section between the first and second chamber (10, 20) and the vacuum chamber (40), respectively. The non-porous sections acts as membranes in the pressure sensors, and will react to pressure changes in the first and second chambers (10, 20).

In an embodiment, the first and/or second pressure sensors (12, 22) are therefore absolute pressure sensors.

In an embodiment the interstitial fluid glucose measuring device (1) comprises a vacuum chamber (40) being a reference for at least one of the first and second pressure sensors (12, 22).

Absolute pressure measurements use the absolute zero, or vacuum, as the reference point; as opposed to gauge measurements used in prior art where ambient pressure is used as the reference. In Fig. 1a, it can be seen that the membranes of the pressure sensors (12, 22) always has vacuum as the reference point, since the membranes interface the vacuum chamber (40) on one side.

The pressure sensors comprises in an embodiment an array of embedded micro electro mechanical (MEMS) switches which closes in turn as the membrane and the support structure is moving relative to each other in response to changing trans-membrane pressure gradients. The MEMS switches would act as a mechanical analogue to digital converter transforming a pressure change directly into a bit stream, or alternatively a series of discrete voltages or resistance changes depending on the applied bias circuitry. Such sensors can be found as off-the-shelf products, based on e.g., a piezo resistive or a capacitive principle. The resolution of the system is in this embodiment limited by the number of switches incorporated into each sensing element, and the number of sensing elements used. However, nano-technology makes it possibility to enhance the resolution of such MEMS arrangements and at the same time reduce size.

The output signal from the pressure sensors, will be sent to an electronic circuit module (not shown), typically implemented on a printed circuit board (PCB) (60).

In an embodiment the electronic the interstitial fluid glucose measuring device (1) comprises a processing unit arranged for calculating a glucose concentration dependent value from the difference between output signals from the first and second absolute pressure sensors (12, 22). For many purposes the glucose concentration dependent value is sufficient for further processing of the signal, either in the device itself or in an external device.

In the case where the absolute or real glucose concentration value is needed, a glucose concentration output value is calculated, in a further embodiment, from the glucose concentration dependent value, based on a predetermined calibration scheme.

The device (1) may also comprise a radio transmitter wherein the differential signal, or the or absolute glucose concentration value signal is transformed to make it suitable for wireless transmission, and sent to an external receiver unit, according to prior art radio technology. Both the coding (protocol) and the frequency is chosen to provide data integrity, security and low power consumption.

The energy for the electronic circuit and the transmitter can either be supplied internally from a battery, or by for instance magnetic induction.

The device (1) comprises in this embodiment a support structure (3) defining the cavities (10, 20, 40). The illustration is meant to illustrate the principle of the device according to the invention, and the support structure (3) may have many equivalent implementations. E.g., the first and second chambers (10, 20) may belong to two separate mechanical structures, two vacuum chambers can be used instead of one, the membranes may be mechanically separated etc. The actual implementation will depend on design choices, such as the use of off-the shelf components and the technology used for micromachining. In this case, the support structure (3) is surface micro machined on top of the substrate (3a).

The output from the pressure sensors are wire bond and accessible from the PCB (60). Depending on the type and number of pressure sensor elements (14, 24), there may be a number of such wire bindings, where only one from each of the first and second chamber (10, 20) has been illustrated.

All components above may be installed in a housing (2) with perforations (2a) allowing the interstitial fluid to be in direct contact with the first and second membranes (11, 21). An O-ring (12b) between the housing and the membrane is also shown.

In an embodiment the pressure sensors (12, 22) are variable capacitors.

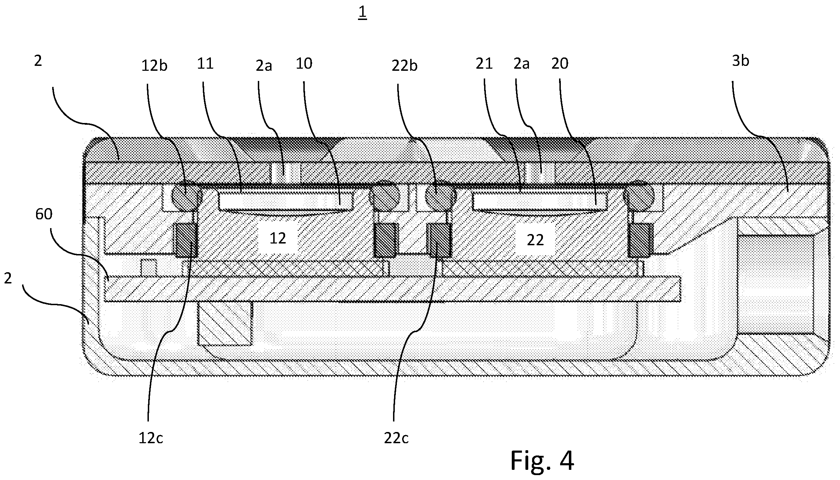

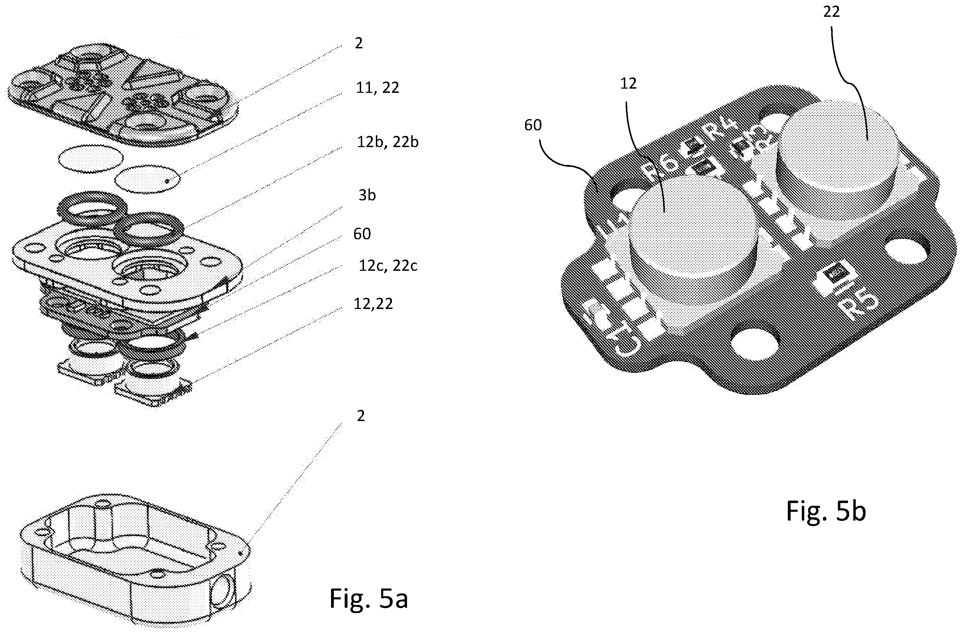

In the illustration in Fig. 1 above, the pressure sensors could be integrated into the structure of the sensor. The cross sectional view of Fig. 4 and the exploded view of Fig. 5a shows another embodiment of the invention, based on pre-fabricated pressure sensors. The description of the structural elements is identical to the description for the embodiment of Fig. 1 above, with the exception that the pressure sensors (12, 22) have been manufactured as separate components that are mounted in a mounting frame (3b), e.g. a metal plate, as part of an assembly process. The housing (2) is here split in a lid and a base. Further, O-rings (12b, 22b) and spacers (12c, 22c) around the pressure sensors (12, 22) are illustrated.

Fig. 5b is a detailed view illustrating the pressure sensors (12, 22) arranged on the PCB (60). The PCB may comprise a processor for processing the output signals from the pressure sensors, as well as a wireless interface.

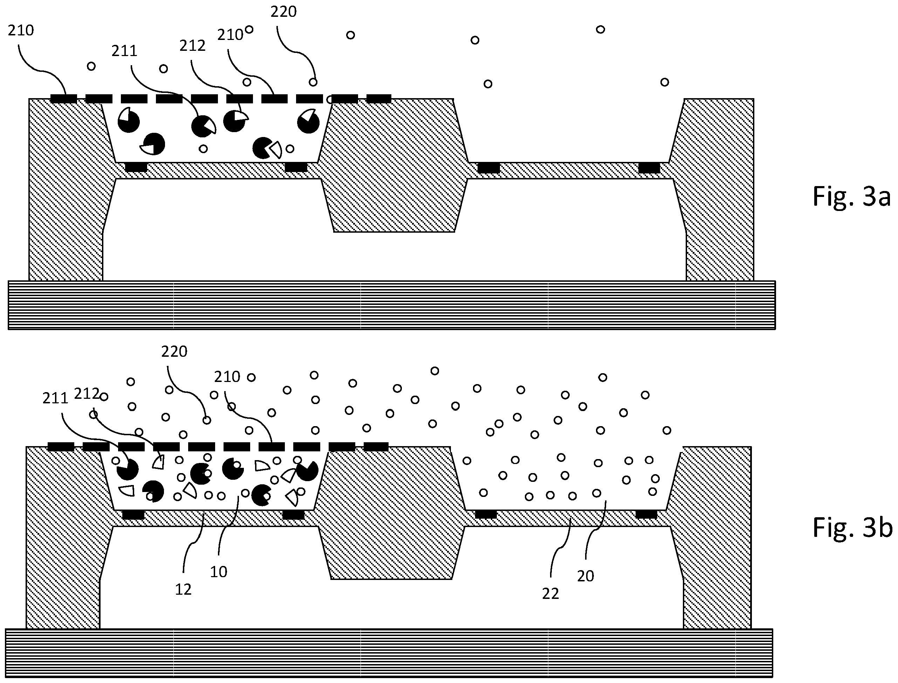

Fig. 3a and 3b illustrate how an increased glucose concentration in the interstitial fluid is detected and measured according to the invention.

As will be explained more thoroughly later, detection of glucose is based on the competition between glucose (220) and a polysaccharide to bind to a receptor, the lectin. Lectin (211) and polysaccharide (212) are present in the reagent chamber in an "active fluid" and enclosed in the first cavity (10) by the nanoporous membrane (210) which is glucose permeable. At low glucose concentration, as can be seen in Fig. 3a, the pressure difference in the reagent chamber, or first chamber (10) is low. Glucose molecules (220) and small ions will pass through the pores of the membrane (210). Glucose molecules (220) which enter the reagent chamber compete with dextran (212) to bind to ConA proteins (211). As the concentration of glucose increases, as illustrated in Fig. 3b, the release of dextran from ConA results in an increase of osmotic pressure. The increased osmotic pressure in the first chamber can then be measured with the first pressure sensor (12). This process is reversible, and a decrease in glucose concentration will cause a reduced pressure.

In addition to the first cavity and the first pressure sensor, a second cavity and a second pressure sensor is also shown. The second pressure sensor will measure all pressure changes that are not related to changes in glucose concentration, such as hydrostatic pressure changes. The signal value from the second pressure sensor can then be used as a basis for determining the changes related to glucose concertation in the first pressure sensor, since the first sensor is also affected by additional pressure changes related to e.g. hydrostatic pressure.

The support structure (3) and the MEMS components can be manufactured by known Silicon micromachining techniques, where microscopic mechanical parts can be fashioned out of a silicon substrate or on a silicon substrate. Both micromachining and surface micromachining, including silicon wafer bonding, can be used.

Fig. 1b and 1c are respective examples of embodiments of the pressure sensors of the measurement device of the invention. Fig. 1b depicts an example of embodiment comprising piezo resistive sensing elements (R1, R2, R3, R4) arranged in a Wheatstone bridge arrangement. Fig. 1c depicts an example of embodiment comprising sensing elements comprising MEMS switches as described above. In both examples of embodiment, the sensing structure (12a, 22a), i.e., the part of the pressure sensor that deforms as a result of applied pressure in the cavity (10, 20), could be made of silicon, such as monocrystalline, polycrystalline or silicon glass. The piezoelectric effect in a monocrystalline semiconductor is caused by compression or stretching of the crystal grid occurring as a result of extremely small mechanical deformations. Thus, the change in resistivity is higher than in prior art sensors, e.g., strain gauges, whose resistance changes with geometrical changes in the structure.

The sensing structure (12a, 22a) is in this example attached to a rigid base substrate of silicon, glass, ceramic or polymer being part of the support structure (3), and to a spacer (3a) made from one of the materials used in the support structure (3).

In some examples of embodiments the base substrate 5, spacer 6, is made from the same piece of material.

In the example of embodiment depicted in Fig. 1b, the piezo resistive sensing elements (R1, R2, R3, R4) are configured as a Wheatstone bridge circuit, in which one pair (e.g. Rl and R4) is related to longitudinal stress component measurements (compressive stress), while the other pair (e.g. R2 and R3) is related to transverse stress component measurements (tensile stress). The differences in the longitudinal and transverse piezo resistance coefficients will then result in one pair increasing their resistance, whereas the second pair will decrease their resistance in response to a pressure change. Alternatively, the piezo resistive elements can be configured as either a single variable resistor or as two variable resistors, respectively. By providing a dimensionless measurement, (i.e. providing a measurement of an expression comprising resistive elements divided by another expression for the same resistive elements, as known to a person skilled in the art, this will cancel noise present in the nominator and denominator of the expression for the dimensionless measurement), for example white noise present in the measurements. The Wheatstone bridge is connected between two terminals V+ and V- as depicted in Fig. 1a providing for example a DC voltage across the bridge enabling the measurements of the resistive changes by measuring the output voltage at the terminals Vo+ and Vo-, as known to a person skilled in the art.

In an example of embodiment of the present invention, an internally located battery or RF induced power from an externally located source powers the Wheatstone bridge. In another example of embodiment of the present invention, a pulsating signal is used to power the Wheatstone bridge, for example from the RF source powering the Wheatstone bridge. The measurements signals from the sensor can be processed in a phase lock (lock in) amplifier that will further cancel noise components and other surrounding RF signals from the measurement signals as known to a person skilled in the art.

The output voltages Vo+ and Vo- can in an embodiment be transmitted wirelessly to an externally located processing unit providing further processing of the measurements, as known to a person skilled in the art.

In another embodiment the output voltages Vo+ and Vo- are converted, in an Analog to Digital converter to a digital signal, e.g. a 24 bit digital signal before being transmitted over a wireless protocol, as known by a person skilled in the art,

In another example of embodiment as depicted in Fig. 1c, the sensing elements Rn comprises MEMS switches arranged in a circuit wherein different respective switches are engaged and are closing (or opening) the circuit with changing strain in the sensing structure as a response to a changing trans-membrane pressure gradient. The switches can for example be arranged as known to a person skilled in the art to form closed circuits resembling binary data conversion in which bit streams of highs (V+) and lows (V-) converts the pressure directly into digital data, omitting the use of sensor driver circuits and analogue to digital converters. The switches can have different weight, e.g. depending on doping or location relative deformation, such that their weight ranges from a MSB (most significant bit) to a LSB (least significant bit).

The switches can be manufactured using traditional MEMS fabrication technologies in which alternating electrically conducting layers or adjacent components are brought in or out of contact as the membrane moves back and forth. Nanofabrication technologies have facilitate the integration of high density switching arrays to a great extent. The sensor device according to the present invention will in its basic configuration measure absolute osmotic pressures.

Glucose molecules in open chain form have a diameter of about 1.5 nm. The glucose permeable membranes should therefore preferably have pore diameter larger than 1.5 nm.

In an embodiment the pore diameter is between 2 and 10 nm.

Further, the membrane can be an asymmetric membrane with an active layer and a base layer, where the diameter indicated above is for the active layer and the pore diameter of the base layer is larger than for the active layer.

Preferably the base layer is mounted towards the first cavity (10).

Since the membrane is intended for implantation in a body, the material should be non-toxic and biocompatible. In an embodiment the first glucose permeable membrane is a ceramic membrane made from monolithic nanoporous anodic aluminum oxide (AAO), as available to the person skilled in the art.

In an embodiment, a semi-permeable membrane is enclosing a part of the second cavity (20). This is preferably a second glucose porous membrane (11) interfacing on one side the interior of said second cavity (20) and on the other side arranged for interfacing the interstitial body fluid. However, since the second cavity does not contain any active fluid, no osmotic pressure change will result from a change in the glucose concentration.

When the two cavities both have glucose permeable membranes, large and small hydrostatic pressure changes as well as small changes in pressure caused by other factors, such as other osmotic contributions, not due to the active solution, that would otherwise appear as noise or measurement errors, can be filtered out by subtracting one signal from the other.

In an embodiment, related to the embodiment above, the first and second cavities (10, 20) and the first and second membranes (12, 22) are identical, respectively. In this situation, no calibration or normalization of the sensor output signals from the first and second sensors is necessary to obtain a filtered output value representing the glucose level.

In an embodiment the invention is a glucose measuring system comprising an interstitial fluid glucose measuring device (1) according to any of the embodiments above and an external device (not illustrated), wherein the interstitial fluid glucose measuring device (1) and the external device both have wireless interfaces, and are arranged to communicate wirelessly.

In an embodiment the system comprises a processor arranged for calculating a glucose concentration dependent value from the difference between output signals from the first and second absolute pressure sensors (12, 22). The processor may be arranged in the measuring device or in the external device. The glucose concentration dependent is a function of the glucose concentration

In a related embodiment an absolute glucose concentration output value is calculated from the glucose concentration dependent value, based on a predetermined calibration scheme.

In an embodiment the modulated wireless signal between the glucose measuring device (1) and the external device is digital. This makes the design more immune to interference and scalable.

Active solution

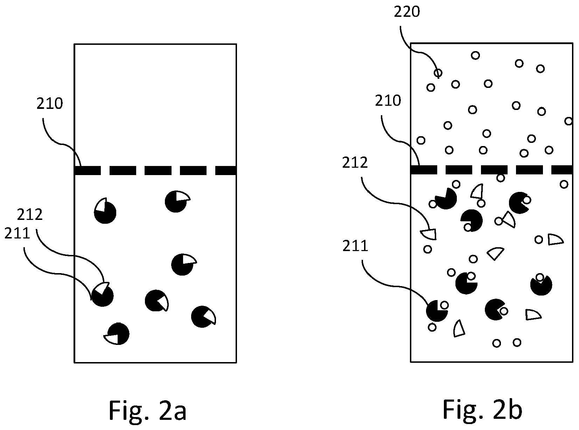

The sensing principle of an implantable glucose sensor relies on osmotic pressure variations between a reagent chamber and the solution, see illustrations in Figures 2a and 2b. Detection of glucose is based on the competition between glucose (220) and a polysaccharide (dextran) to bind to a receptor, the lectin Concanavalin A (ConA). ConA (211) and dextran (212) are present in the reagent chamber in an "active fluid" and are not to exit through the nanoporous membrane (210). At low glucose concentration, as shown in Fig. 2a, the pressure difference in the reagent chamber is low. Glucose molecules and small ions will pass through the pores of the membranes. Glucose molecules which enter the reagent chamber compete with dextran to bind to ConA proteins. As the concentration of glucose increases, Fig. 2b, the release of dextran from ConA results in an increase of osmotic pressure that can be used to quantify the concentration of glucose. This process is reversible and as the glucose concentrations falls, the ConA reattaches back to the dextran forming a large macromolecular complex from the ConA and dextran "sub units". The corresponding decrease in the number of free particles triggers the osmotic pressure to fall.

The osmotic pressure Π of an ideal solution of low concentration can be approximated using the Morse equation:

$$\pi = iMRT$$

where i is the dimensionless Van't Hoff factor, M is the molarity, R the gas constant and T the temperature of the chamber.

Fig. 2 shows the linear correlation between osmotic pressures and glucose levels, accurate also at hypo and hyper glycemic levels.

The inventors found that lowering the viscosity of the composition would consequently reduce the asymmetry and response time of the sensor. In order to diminish the viscosity of the active fluid, the following parameters were explored while varying the glucose concentration in the solutions:

- Molecular weight of the dextran (10kDa, 40kDa or 70kDa)

- Ratio of ConA to dextran

- Initial concentration of ConA.

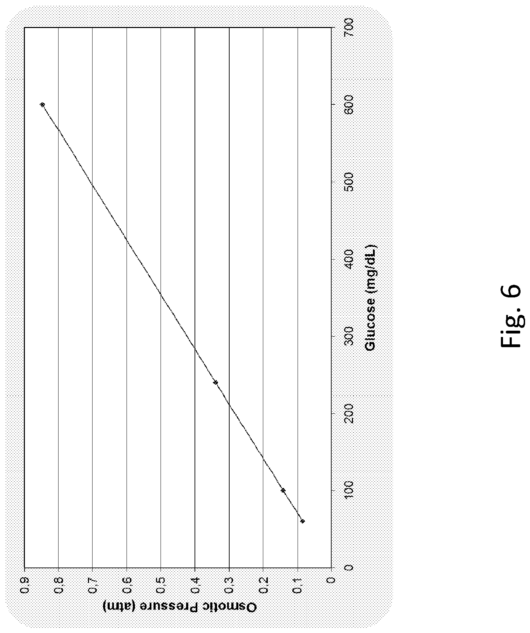

Fig. 6 shows viscosity as a function of glucose concentration for dextran 10kDa, 40kDa and 70kDa, the molar ratio between ConA to dextran was 6:1, using initial concentrations respectively 3 mM and 0.5 mM. This active fluid composition was considered as a "baseline" for the viscosity measurements.

Lowering the ConA concentration and more specifically, the ConA to dextran concentrations ratio, was expected to decrease the number of intermolecular bonds between dextran and ConA, thus lowering the viscosity of the system. To investigate this effect, the ConA to dextran was reduced from 6:1 to 3:1, using the following concentrations ConA 1.5 mM, dextran 0.5 mM and glucose range 2 to 30 mM.

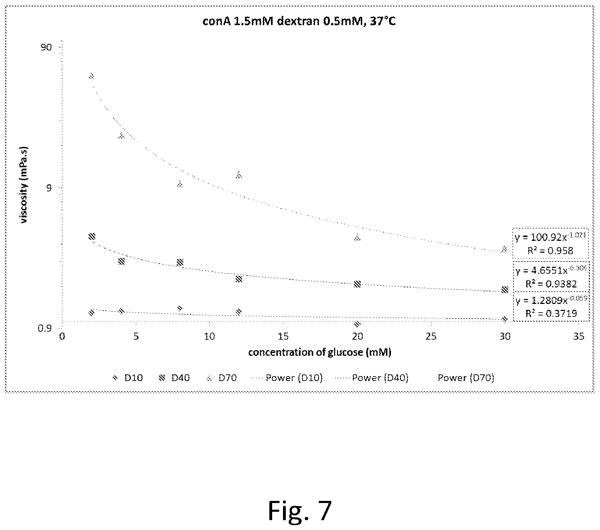

Fig. 7 clearly shows that the viscosity of each system is decreased by one to two orders of magnitude when the ConA concentration is divided by two (all other parameters are kept constant). This is expected to have an extremely significant effect on the response time and the asymmetry of the response with increasing or decreasing glucose concentration.

Decreasing the molar ratio of ConA to dextran to 1:1 was shown to improve the amplitude of the response of the sensor. Lowering the number of intermolecular bonds between ConA and dextran also lowered the viscosity. The inventors chose to test a slightly higher concentration of dextran (1 mM instead of 0.5 mM) since it was also shown to improve the amplitude of the response and the sensitivity of the sensor. An active fluid with a ConA and dextran concentrations of 1 mM showed an amplitude of response approximately three times larger than the amplitude of response of the "baseline" active fluid described above.

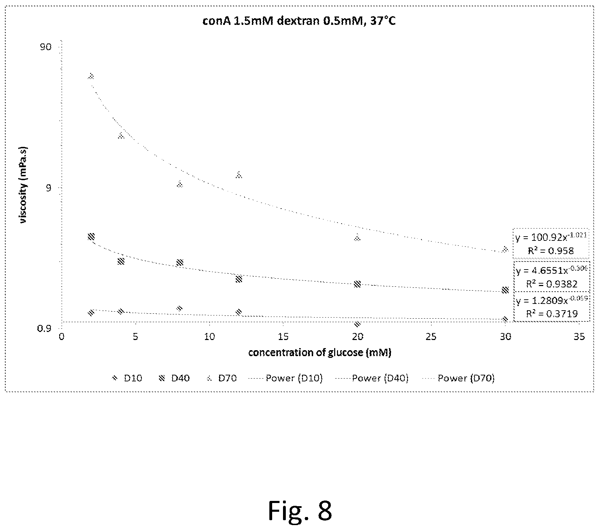

By keeping the molar concentrations of dextran and ConA equal (at 1 mM), the viscosity was further decreased by an order of magnitude when compared to 3:1 molar ratio of ConA to dextran, see Fig. 8. Furthermore, the influence of the glucose concentration on viscosity is lessened. This decreased the asymmetry of the response times to ascending and descending glucose concentrations.

For characterizations, each solution was stirred during 24 hours before viscosity measurements. A Brookfield DV-II+Pro viscometer was used for all measurements. The viscometer drives a spindle through a calibrated spring which is immersed in the active fluid. The viscous drag of the fluid against the spindle is measured by the spring deflection, which is measured with a rotary transducer. The measurement range is determined by the rotational speed of the spindle, the size and shape of the spindle, the container where the spindle is rotating in, and the full scale torque of the calibrated spring. The viscosity appears in units of centipoise (shown "cP"). One centipoise is equal to 1 mPa.s in USI.

To control the temperature (T) at which the measurements were done, the viscometer was equipped with a water bath which can be set at the chosen T. All the viscosity measurements were performed at 37 °C, with a 5 min. waiting period to ensure stabilisation of the temperature in the active fluid.

Based on viscosity measurements and simulation results, the inventors found clear trends in term of sensitivities and kinetics of the sensor can be established:

- A 1 to 1 molar concentration ratio of ConA to dextran was advantageous with respect to the sensitivities in term of osmotic pressure in the range of glucose concentration [0;30] mM.

- A 1 to 1 molar concentration ratio of ConA to dextran was advantageous with respect to fast kinetics and the least asymmetrical response to glucose variations since the viscosity of the active fluid varied very little with the glucose concentration.

- At 1 to 1 molar concentration ratio of ConA to dextran, a high ConA concentration (=dextran concentration) gave better sensitivity. However, it was also likely to increase the viscosity of the system, in turn increasing the time response of the sensor.

- Experimental studies of the viscosities of different active fluids were performed. These showed a rapid drop (of 3 orders of magnitude) as the concentration of ConA was decreased from 3 mM (based on monomer concentration) to 1 mM. Changes in the concentration of dextran also influenced the viscosity but to a lesser extent. In contrast the molecular weight of the dextran (10kDa, 40kDa, 70kDa) could change the viscosity by orders of magnitude, see figures 6 and 7.

- The lower the MW of the dextran, the lower the viscosity was. This means that an active fluid containing dextran 10kDa will give a faster response than one containing dextran 40kDa, which in turn is expected to give a faster response than one containing dextran 70kDa.

- Higher MW of the dextran, provided higher differential osmotic pressure of the system. Using a higher size dextran (e.g. 70kDa) in the active fluid is therefore going to improve the sensitivity of the system.

- Simulations of the response time based on these measurements indicated that number of active fluids are expected to have response times (in both increasing a decreasing glucose concentrations) of less than 5 minutes.

The inventors performed a number of experiments to test the response of different active fluids, e.g.:

- 1.0 mM ConA, 1.0 mM dextran 40 kDa and 1.0 mM ConA, 1.0 mM dextran 70 kDa.

- 1.5 mM ConA, 1.5 mM dextran 40 kDa and 1.5 mM ConA, 1.5 mM dextran 70 kDa.

Test of active fluids based on 1.5 mM ConA, 1.5 mM dextran 40 kDa

Experiments were carried out by cycling the contents of the sample chamber in a macrocell between 2 mM glucose (in a solution containing 100 mM Trizma B buffer, 150 mM NaCl, 10mM MgCl2 and 10mM CaCl2) and 30 mM glucose in the same solution. At each change of glucose concentration, the solution was removed by hand using a pipette, taking great care not to touch the nanoporous (NP) membrane but bringing the pipette tip as close as possible to the bottom of the sample chamber. The solution was then replaced by the new chosen solution, which was agitated by "pumping" the pipette. The solution was removed and replaced by fresh solution another two times. Temperature was 21 °C.

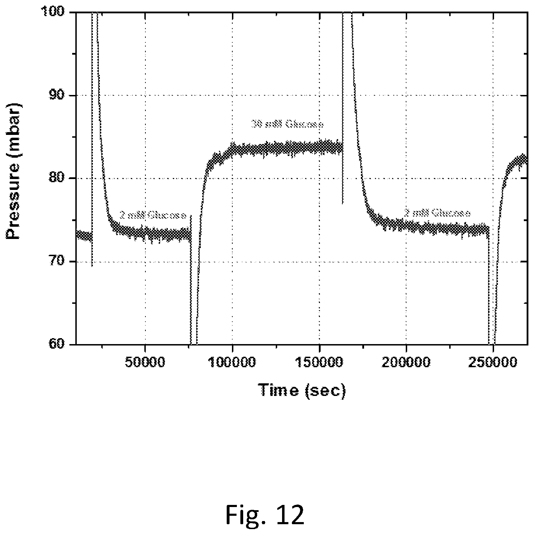

Following this procedure, a reproducible signal was obtained. A change from 2 mM glucose to 30 mM glucose resulted in a large spike down in the measured pressure, followed by a slow rise to a new pressure that was approximately 10 mbar higher than the original value. A change from 30 mM glucose to 2 mM glucose gave a large spike up in measured pressure followed by a slow drift downwards (see Fig. 12). Both the fast spike in one direction and the slow drift in the opposite direction occurred each time.

These two features can be understood when we consider the time course of the events taking place in sample chamber and active fluid chamber, as described below.

1) Starting at low glucose concentration, to begin with, all small solutes are present in equal concentrations on both sides of the NP membrane. Most of the ConA and dextran inside the active fluid chamber is bound together, giving a small overpressure inside the chamber.

2) Glucose is added outside the NP membrane. The concentration of solutes is briefly higher outside the membrane than inside. There is a brief underpressure inside the chamber.

3) The glucose diffuses through the membrane. Small solutes are present in equal concentrations on both sides of the NP membrane. ConA and dextran dissociate and the measured pressure increases to finally give an overpressure inside the chamber.

Thus the observed spike is not an artifact, but shows the process of equilibration of the glucose concentration on the two sides of the membrane.

Results of the study on the active fluids also shows that the present active fluid compositions could be studied for 3 months with no perceptible loss of sensitivity, i.e. long term stability.

The following section describes different embodiments of the active fluid solution being part of the invention. These embodiments can individually be combined with all the embodiments of the structural elements of the sensor described above.

In a first aspect the present invention provides an active fluid composition for use as an active fluid in a continuous glucose sensor comprising a carbohydrate-binding molecule, the carbohydrate-binding molecule being a lectin; a lectin-binding molecule, the lectin-binding molecule being a polysaccharide; at least one chloride salt of a divalent metal ion, and optionally glucose, wherein the said lectin to said polysaccharide molar ratio may be from 3:1 to 1:1.

In a first embodiment the lectin to polysaccharide molar ratio in the said composition is from 2:1 to 1:1. In a second embodiment the lectin to polysaccharide molar ratio in the said composition is 3:1, 2:1 or 1:1.

In a third embodiment the polysaccharide in the said composition may be dextran or other polysaccharide, having a molecular weight 10-100 kDa, e.g. 10-70 kDa. The dextran may have a molecular weight of 10 kDa, 40 kDa or 70 kDa.

In a fourth embodiment the concentration of said lectin in the said composition may be 0.2-5 mM, 0.5-3 mM or more specific 1-1.5 mM.

In a fifth embodiment the lectin in the said composition is Concanavalin A (ConA).

In a sixth embodiment the concentration of the polysaccharide in the said composition is 0.2-5 mM, 0.5-3 mM or more specific 1-1.5 mM.

In a seventh embodiment the concentration of ConA in the composition is 0.2-5 mM, more specific 0.5-3 mM, 1-1.5 mM, 1.5 mM or 1 mM based on the monomer concentration, and the concentration of dextran is 0.2-5 mM, more specific 0.5-3 mM, 1-1.5 mM, 1.5 mM or 1 mM. In an eight embodiment the ConA concentration in the composition is 1 mM based on the monomer concentration, and the dextran concentration is 1mM. In a ninth embodiment the said ConA concentration is 1.5 mM based on the monomer concentration, and the dextran concentration is 1.5 mM.

In a tenth embodiment the composition, according to any of the above embodiments, may further comprise an aqueous buffer solution with pH 7.0-7.8. The buffer may be a Tris buffer or HEPES.

In a specific embodiment according to the tenth embodiment, the aqueous buffer solution contains 10-120 mM, for instance 100 mM, of a Tris buffer. In another specific embodiment according to the tenth embodiment the aqueous buffer solution in said composition contains 1-40 mM, or 1-20 mM, of HEPES buffer. The aqueous buffer solution, according to the tenth embodiment, may have a pH 7.2-7.6, for instance 7.35 - 7.5 or 7.4-7.5.

Verification of pH in the buffer solution may be performed at room temperature or 37 $\underline{o}$C using a reference pH meter. Other temperatures may also be utilized depending on the intended use of the composition.

In an eleventh embodiment of the said composition, according to any of the above embodiments, the at least one chloride salt of a divalent metal ion is chosen from MgCl2, CaCl2 and MnCl2. In a specific embodiment of the eleventh embodiment, the at least one chloride salt of a divalent metal ion, or a combination thereof is dissolved in the said aqueous buffer solution giving concentrations 1-10 mM MgCl2, 1-10 mM CaCl2 and 1-10 mM MnCl2. The aqueous solution may also comprise NaCl giving an isotonic solution.

In a specific embodiment according to the eleventh embodiment, the said aqueous buffer solution comprises at least one of 10 mM MgCl2, 10 mM CaCl2 and 10 mM MnCl2 or a combination thereof and 150 mM NaCl, and optionally 20-40 mM glucose. In another specific embodiment according to the eleventh embodiment, the aqueous buffer solution contains 10 mM MgCl2, 10 mM CaCl2,150 mM NaCl, and 30 mM glucose. The presence of glucose minimizes ConA-dextran binding, keeping the viscosity of the solution low and making handling easier.

In the composition to the present invention, according to any of the above embodiments, the water used in the aqueous buffer solution is reverse osmosis water, deionized water or distilled water.

In a specific embodiment a composition according to present invention has the following composition

- 1.0 mM or 1.5 mM ConA based on the monomer concentration,

- 1.0 mM or 1.5 mM dextran 40 or dextran 70,

- Tris buffer 100 mM, pH 7.4-7.5,

- 10 mM MgCl2, 10 mM CaCl2 and 150 mM NaCl,

- 30 mM glucose

wherein the said lectin to said polysaccharide molar ratio is 1:1.

In an embodiment, that can be related to any of the embodiment structural elements of the device, the first and second cavities (12, 22) comprise similar buffer solutions.

In the exemplary embodiments, various features and details are shown in combination. The fact that several features are described with respect to a particular example should not be construed as implying that those features by necessity have to be included together in all embodiments of the invention. Conversely, features that are described with reference to different embodiments should not be construed as mutually exclusive. As those with skill in the art will readily understand, embodiments that incorporate any subset of features described herein and that are not expressly interdependent have been contemplated by the inventor and are part of the intended disclosure. However, explicit description of all such embodiments would not contribute to the understanding of the principles of the invention, and consequently some permutations of features have been omitted for the sake of simplicity or brevity.

**Claims**

1. An interstitial fluid glucose measuring device (1), comprising:

- first and second cavities (10, 20), both configured for being in fluid communication with interstitial fluid outside the cavities, wherein the first and second pressure sensors (12 , 22) are absolute pressure sensors configured for sensing a pressure in said first and second cavities (10, 20), respectively, wherein

    a) the first cavity (10) comprises an active solution and is defined in part by a first glucose porous membrane (11) interfacing on one side the interior of said first cavity (10) and on the other side configured for interfacing the interstitial body fluid, and wherein the active solution (13) is a composition comprising a lectin and a polysaccharide wherein the lectin is Concanavalin A (ConA) and the polysaccharide is dextran and wherein the ConA to dextran ratio is from 3:1 to 1:1 and the dextran has a molecular weight of 40 kDa or 70 kDa;
    b) the second cavity (20) not comprising said active solution and defined in part by a second glucose porous membrane (21) interfacing on one side the interior of said second cavity (20) and on the other side arranged for interfacing the interstitial body fluid.

2. The interstitial fluid glucose measuring device (1) of claim 1, wherein the first and second cavities (12, 22) comprise similar buffer solutions.

3. The interstitial fluid glucose measuring device (1) of claim 1 or 2, wherein the pore diameter of the first and second glucose porous membrane (11, 21) are between 2 and 10 nm.

4. The interstitial fluid glucose measuring device (1) of anyone of claim 1 to 3, wherein the first and second cavities (10, 20) are identical and the first and second membranes (11, 21) are identical.

5. The interstitial fluid glucose measuring device (1) according to anyone of the claims 1 to 4, wherein the first pressure sensor (12) is arranged opposite the first glucose porous membrane (11) in the first cavity (10).

6. The interstitial fluid glucose measuring device (1) of claim 5, comprising a vacuum chamber (40) being a reference for at least one of the first and second pressure sensors (12, 22).

7. A method for measuring an interstitial fluid glucose level, comprising the steps of;

    - allowing glucose from the interstitial fluid through a first glucose porous membrane (11) and into a first cavity (10) comprising an active solution comprising a lectin and a polysaccharide wherein the lectin is Concanavalin A (ConA) and the polysaccharide is dextran and wherein the ConA to dextran ratio is from 3:1 to 1:1 and the dextran has a molecular weight of 40 kDa or 70 kDa;
- passing glucose from the interstitial fluid through the second glucose porous membrane (21) into a second cavity (20);- measuring pressure in the first and second cavity (10, 20) by first and second absolute pressure sensors (12, 22) respectively,
- calculating a glucose concentration dependent value from the difference between output signals from the first and second absolute pressure sensors (12, 22).

8. The method for measuring an interstitial fluid glucose level of claim 7, wherein the first and second glucose porous membrane (11, 21) have a pore diameter between 2 and 10 nm.

9. The method for measuring an interstitial fluid glucose level of claims 7 or 8, comprising the step of calculating a glucose concentration output value from the glucose concentration dependent value, based on a predetermined calibration scheme.

10. A glucose measuring system comprising an interstitial fluid glucose measuring device (1) according to any of the claims 1 to 6 and an external device, wherein the interstitial fluid glucose measuring device (1) and the external device both have wireless interfaces, and are arranged to communicate wirelessly.

**Patentansprüche**

1. Vorrichtung (1) zur Messung von Glucose in interstitieller Flüssigkeit, umfassend:

    - einen ersten und zweiten Hohlraum (10 , 20), die beide so ausgelegt sind, dass sie außerhalb der Hohlräume mit der interstitiellen Flüssigkeit in Flüssigkeitsverbindung stehen, wobei der erste und zweite Drucksensor (12 , 22) Absolutdrucksensoren sind, die zum Erfassen eines Drucks in dem ersten bzw. zweiten Hohlraum (10 , 20) ausgelegt sind, wobei

    a) der erste Hohlraum (10) eine aktive Lösung umfasst und teilweise durch eine erste poröse Glucosemembran (11) definiert ist, die auf einer Seite die Schnittstelle für das Innere des ersten Hohlraums (10) aufweist und auf der anderen Seite so ausgelegt ist, dass sie eine Schnittstelle für die interstitielle Körperflüssigkeit aufweist, und wobei die aktive Lösung (13) eine ein Lectin und ein Polysaccharid umfassende Zusammensetzung ist, wobei das Lectin Concanavalin A (ConA) ist und das Polysaccharid Dextran ist, und wobei das Verhältnis von ConA zu Dextran von 3:1 bis 1:1 beträgt und das Dextran ein Molekulargewicht von 40 kDa oder 70 kDa aufweist;

b) der zweite Hohlraum (20) die aktive Lösung nicht umfasst und teilweise durch eine zweite poröse Glucosemembran (21) definiert ist, die auf einer Seite die Schnittstelle für das Innere des zweiten Hohlraums (20) aufweist und auf der anderen Seite so angeordnet ist, dass sie eine Schnittstelle für die interstitielle Körperflüssigkeit aufweist.

2. Vorrichtung (1) zur Messung von Glucose in interstitieller Flüssigkeit nach Anspruch 1, wobei der erste und zweite Hohlraum (12, 22) ähnliche Pufferlösungen umfassen.

3. Vorrichtung (1) zur Messung von Glucose in interstitieller Flüssigkeit nach Anspruch 1 oder 2, wobei der Porendurchmesser der ersten und zweiten porösen Glucosemembran (11, 21) zwischen 2 und 10 nm liegt.

4. Vorrichtung (1) zur Messung von Glucose in interstitieller Flüssigkeit nach einem der Ansprüche 1 bis 3, wobei der erste und zweite Hohlraum (10, 20) identisch sind, und die erste und zweite Membran (11, 21) identisch sind.

5. Vorrichtung (1) zur Messung von Glucose in interstitieller Flüssigkeit nach einem der Ansprüche 1 bis 4, wobei der erste Drucksensor (12) gegenüber der ersten porösen Glucosemembran (11) im ersten Hohlraum (10) angeordnet ist.

6. Vorrichtung (1) zur Messung von Glucose in interstitieller Flüssigkeit nach Anspruch 5, umfassend eine Unterdruckkammer (40), die eine Referenz für mindestens einen des ersten und zweiten Drucksensors (12, 22) darstellt.

7. Verfahren zur Messung eines Glucosespiegels in der interstitiellen Flüssigkeit, umfassend die folgenden Schritte;

- Zulassen von Glucose von der interstitiellen Flüssigkeit durch eine erste poröse Glucosemembran (11) und in einen ersten Hohlraum (10) umfassend eine aktive Lösung umfassend ein Lectin und ein Polysaccharid, wobei das Lectin Concanavalin A (ConA) ist und das Polysaccharid Dextran ist, und wobei das Verhältnis von ConA zu Dextran von 3:1 bis 1:1 beträgt, und das Dextran ein Molekulargewicht von 40 kDa oder 70 kDa aufweist;

- Durchleiten von Glucose von der interstitiellen Flüssigkeit durch die zweite poröse Glucosemembran (21) in einen zweiten Hohlraum (20);- Messen des Drucks in dem ersten und zweiten Hohlraum (10, 20) durch einen ersten bzw. zweiten Absolutdrucksensor (12, 22),

- Berechnen eines von der Glucosekonzentration abhängigen Wertes aus der Differenz zwischen Ausgangssignalen von dem ersten und zweiten Absolutdrucksensor (12, 22).

8. Verfahren zur Messung eines Glucosespiegels in der interstitiellen Flüssigkeit nach Anspruch 7, wobei die erste und zweite poröse Glucosemembran (11, 21) einen Porendurchmesser zwischen 2 und 10 nm aufweisen.

9. Verfahren zur Messung eines Glucosespiegels in der interstitiellen Flüssigkeit nach Anspruch 7 oder 8, umfassend den Schritt des Berechnens eines Glucosekonzentrationsausgangswertes aus dem von der Glucosekonzentration abhängigen Wert basierend auf einem vorbestimmten Kalibrierungsschema.

10. System zur Messung von Glucose umfassend eine Vorrichtung (1) zur Messung von Glucose in der interstitiellen Flüssigkeit nach einem der Ansprüche 1 bis 6 und eine externe Vorrichtung, wobei die Vorrichtung (1) zur Messung von Glucose in der interstitiellen Flüssigkeit und die externe Vorrichtung beide Drahtlosschnittstellen aufweisen und zum drahtlosen Kommunizieren vorgesehen sind.

**Revendications**

1. Dispositif de mesure du glucose dans un fluide interstitiel (1), comprenant :

- des première et deuxième cavités (10, 20), toutes deux étant configurées pour être en communication fluidique avec le fluide interstitiel à l'extérieur des cavités, dans lequel les premier et deuxième capteurs de pression (12, 22) sont des capteurs de pression absolue configurés pour détecter une pression respectivement dans lesdites première et deuxième cavités (10, 20), dans lequel

a) la première cavité (10) comprend une solution active et est définie en partie par une première membrane poreuse de glucose (11) interfaçant d'un côté l'intérieur de ladite première cavité (10) et de l'autre côté configurée pour interfacer le fluide corporel interstitiel, et dans lequel la solution active (13) est une composition comprenant une lectine et un polysaccharide, dans lequel la lectine est la concanavaline A (ConA) et le polysaccharide est le dextrane, et dans lequel le rapport de ConA au dextrane est de 3:1 à 1:1, et le dextrane présente un poids moléculaire de 40 kDa ou de 70 kDa ;

b) la deuxième cavité (20) ne comprend pas ladite solution active et est définie en partie par une deuxième membrane poreuse de glucose (21) interfaçant d'un côté l'intérieur de ladite deuxième cavité (20) et de l'autre côté agencée pour interfacer le fluide corporel interstitiel.

2. Dispositif de mesure du glucose dans un fluide interstitiel (1) selon la revendication 1, dans lequel les première et deuxième cavités (12, 22) comprennent des solutions tampons similaires.

3. Dispositif de mesure du glucose dans un fluide interstitiel (1) selon la revendication 1 ou 2, dans lequel le diamètre des pores des première et deuxième membranes poreuses de glucose (11, 21) est compris entre 2 et 10 nm.

4. Dispositif de mesure du glucose dans un fluide interstitiel (1) selon l'une quelconque des revendications 1 à 3, dans lequel les première et deuxième cavités (10, 20) sont identiques, et les première et deuxième membranes (11, 21) sont identiques

5. Dispositif de mesure du glucose dans un fluide interstitiel (1) selon l'une quelconque des revendications 1 à 4, dans lequel le premier capteur de pression (12) est disposé en face de la première membrane poreuse de glucose (11) dans la première cavité (10).

6. Dispositif de mesure du glucose dans un fluide interstitiel (1) selon la revendication 5, comprenant une chambre à vide (40) servant de référence pour au moins l'un des premier et deuxième capteurs de pression (12, 22).

7. Procédé pour mesurer un niveau de glucose dans un fluide interstitiel, comprenant les étapes consistant à ;

- permettre au glucose du fluide interstitiel à travers une première membrane poreuse de glucose (11) et dans une première cavité (10) comprenant une solution active comprenant une lectine et un polysaccharide, dans lequel la lectine est la concanavaline A (ConA) et le polysaccharide est le dextrane, et dans lequel le rapport de ConA au dextrane est de 3:1 à 1:1, et le dextrane présente un poids moléculaire de 40 kDa ou de 70 kDa ;

- faire passer le glucose du fluide interstitiel par la deuxième membrane poreuse de glucose (21) dans la deuxième cavité (20) ;

- mesurer la pression dans la première et la deuxième chambre (10, 20) respectivement par des premier et deuxième capteurs de pression absolue (12, 22),

- calculer une valeur dépendante de la concentration de glucose à partir de la différence entre les signaux de sortie des premier et deuxième capteurs de pression absolue (12, 22).

8. Procédé pour mesurer un niveau de glucose dans un fluide interstitiel selon la revendication 7, dans lequel les première et deuxième membranes poreuses de glucose (11, 21) présentent un diamètre de pore compris entre 2 et 10 nm.

9. Procédé pour mesurer un niveau de glucose dans un fluide interstitiel selon la revendication 7 ou 8, comprenant l'étape de calcul d'une valeur de sortie de concentration de glucose à partir de la valeur dépendante de la concentration de glucose, sur la base d'un schéma d'étalonnage prédéterminé.

10. Système de mesure du glucose comprenant un dispositif de mesure du glucose dans un fluide interstitiel (1) selon l'une quelconque des revendications 1 à 6 et un dispositif externe, dans lequel le dispositif de mesure du glucose dans un fluide interstitiel (1) et le dispositif externe ont tous deux des interfaces sans fil, et sont agencés pour communiquer sans fil.

**Fig. 1a**

**Fig. 1b**

**Fig. 1c**

Fig. 2b

Fig. 2a

Fig. 3a

Fig. 3b

Fig. 4

EP 3 634 214 B1

Fig. 5b

Fig. 5a

Fig. 6

Fig. 7

**conA 1.5mM dextran 0.5mM, 37°C**

$y = 100.92x^{-1.021}$
$R^2 = 0.958$

$y = 4.6551x^{-0.306}$
$R^2 = 0.9382$

$y = 1.2809x^{-0.059}$
$R^2 = 0.3719$

◈ D10    ▩ D40    ▵ D70    ······ Power (D10)    ······ Power (D40)    ······ Power (D70)

Fig. 8

Fig. 9

Fig. 10

Fig. 11

Fig. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1631187 B1
- US 4822336 A
- US 5337747 A
- WO 2009025563 A1